# EUROPEAN PATENT SPECIFICATION

(11) **EP 4 167 921 B1**
(45) Date of publication and mention of the grant of the patent: **11.10.2023**
(21) Application number: 21731818.7
(22) Date of filing: 15.06.2021
(51) Int. Cl.: A61K 8/02, A61K 8/34, A61K 8/41, A61K 8/891, A61K 8/898, A61Q 5/12, A61Q 5/00

(54) **HAIR CONDITIONING COMPOSITION FOR IMPROVED DEPOSITION**
HAARPFLEGEZUSAMMENSETZUNG ZUR VERBESSERTEN ABSCHEIDUNG
COMPOSITION D'APRÈS-SHAMPOOING POUR DÉPÔT AMÉLIORÉ

(30) Priority: 19.06.2020 EP 20181255
(43) Date of publication of application: 26.04.2023
(73) Proprietor: Unilever IP Holdings B.V., 3013 AL Rotterdam (NL); Unilever Global IP Limited, Wirral, Merseyside CH62 4ZD (GB)
(72) Inventor: BARFOOT, Richard, Jonathan, Wirral Merseyside CH63 3JW (GB); COOKE, Michael, James, Wirral Merseyside CH63 3JW (GB); MENDOZA FERNANDEZ, Cesar, Ernesto, Wirral Merseyside CH63 3JW (GB); PRICE, Paul, Damien, Wirral Merseyside CH63 3JW (GB)
(74) Representative: Chisem, Janet
(86) International application number: PCT/EP2021/066153
(87) International publication number: WO 2021/255050

(56) References cited:
- WO-A1-03/094872
- WO-A1-2015/075063
- WO-A1-2018/228898
- WO-A2-2007/149248
- US-A1- 2017 281 522

## Description

### Field of the Invention

The invention is concerned with conditioning compositions for the treatment of hair, containing a combination of primary and secondary surfactants having linear alkyl groups of different chain length comprising at least one ester group, and a benefit agent to be deposited onto the hair during use and particularly relates to a conditioning composition that enables increased amounts of benefit agent to be deposited.

### Background and Prior art

In personal care compositions, such as hair treatment compositions, the deposition and delivery of benefit agents are often key drivers of product performance. For example, many of the hair conditioner products in the market today work to deliver benefits to hair by depositing benefit agents such as fragrance materials, silicones and damage repair actives onto the hair during the wash and care process.

However, consumers report being disappointed by the level of benefit derived from use of some compositions. This is usually caused by insufficient amount of benefit agents being delivered to the surface. It is, therefore, desirable to develop compositions that provide improved delivery of benefit materials to a surface, for example hair.

Various types of cationic compounds are known in hair treatment compositions for a variety of benefits.

WO 17/172117 discloses a composition for treating keratinous substrates comprising a cationic agent comprising a defined first quaternary ammonium compound and an imidazoline compound, a modified starch, two silane compounds, a cationic vinylpyrrolidone polymer and water. Hair treated with the compositions is purported to have improved mass, body, volume, to be easily rinsed, to dry fast, to stay clean longer and be sufficiently conditioned. US 2005/175569 discloses cosmetic compositions, for example for conditioning and styling hair, comprising a cationic surfactant, which may be a quaternary ammonium salt.

JP 2005-060271 discloses an aqueous hair cosmetic composition that can comprise (A) a dimethylpolysiloxane represented by general formula (1), (B) a dimethylpolysiloxane represented by general formula (2), (C) a cyclic dimethylpolysiloxane represented by general formula (3) at a ratio of [(B)+(C)]/(A) greater than or equal to 1; and (D) an additional quaternary ammonium component. The composition is said to provide a range of conditioning benefits to hair in the wet, rinse and dry stages.

Our own published applications WO 02/102334 and WO 01/43718 provide aqueous hair treatment compositions having cleansing and conditioning properties that comprise quaternary ammonium based cationic surfactants having defined hydrocarbyl chains.

Whilst cationic materials are known in home and personal care products, there is a persistent need to provide improved deposition of benefit agents onto hair.

Despite the prior art, there remains a need to deliver improved delivery of benefits to hair without compromising on consumer desired viscosity characteristics.

We have now surprisingly found that compositions comprising a combination of cationic conditioning primary surfactants with cationic co-surfactants, each having a linear alkyl chain of defined length provide an unexpectedly large enhancement in the deposition of benefit agents whilst maintaining excellent product rheology.

All percentages quoted herein are by weight based on total weight, unless otherwise stated. All amounts quoted herein are based on 100 % activity of materials, unless otherwise stated.

### Definition of the Invention

Accordingly, there is provided a composition comprising:
(i) 0.01 to 10 wt % of a linear cationic conditioning primary surfactant; selected from structure 1 and mixtures thereof: 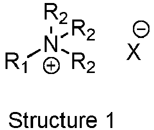 wherein:
   - R₁ comprises a linear alkyl chain having a carbon-carbon chain length of from C₁₆ to C₂₄, preferably C₁₈ to C₂₂;
   - R₂ comprises a proton or a linear alkyl chain having a carbon-carbon chain length of from C₁ to C₄, preferably C₁ to C₂ or a benzyl group; and
   - X is an organic or inorganic anion;
(ii) 0.1 to 10 wt % of a linear fatty material;
(iii) a particulate benefit agent selected from conditioning actives and mixtures thereof; and
(iv) 0.01 to 5 wt % of a linear cationic co-surfactant, selected from structure 2 and mixtures thereof 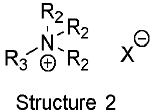 wherein:
   - R₂ comprises a proton or a linear alkyl chain having a carbon-carbon chain length of from C₁ to C₄, preferably C₁ to C₂ or a benzyl group;
   - R₃ comprises a linear alkyl chain comprising an ester group and having an atom-atom chain length of from 3 to 15, preferably 10 to 14; and
   - X is an organic or inorganic anion;

wherein the carbon-carbon chain length of R₁ in structure 1 differs from the atom-atom chain length of R₃ in structure 2 by at least 3 atoms, such that the atom-atom chain length of R₁ in structure 1 is longer than the atom-atom chain length of R₃ in structure 2; and
wherein the molar ratio of linear cationic co-surfactant (iv) to linear cationic conditioning primary surfactant (i) is in the range of from 1:20 to 1:1.

In a second aspect, the invention provides a method of increasing deposition of a particulate benefit agent selected from conditioning actives, preferably silicone emulsion and mixtures thereof to hair comprising the step of applying to hair a composition of the first aspect.

The method of the invention preferably comprises an additional step of rinsing the composition from the hair.

Preferably, the method is a method of increasing silicone deposition to hair comprising the steps of applying to hair a composition as defined by the first aspect of the invention comprising silicone emulsion and rinsing the hair with water.

Compositions in accordance with the invention are preferably formulated as conditioners for the treatment of hair (typically after shampooing) and subsequent rinsing.

### General description of the invention

Preferably, the treatment composition is selected from a rinse-off hair conditioner, a hair mask, a leave-on conditioner composition, and a pre-treatment composition, more preferably selected from a rinse-off hair conditioner, a hair mask, a leave-on conditioner composition, and a pre-treatment composition, for example an oil treatment, and most preferably selected from a rinse-off hair conditioner, a hair mask and a leave-on conditioner composition. The treatment composition is preferably selected from a rinse-off hair conditioner and a leave-on conditioner.

Rinse off conditioners for use in the invention are conditioners that are typically left on wet hair for 1 to 2 minutes before being rinsed off.

Hair masks for use in the present invention are treatments that are typically left on the hair for 3 to 10 minutes, preferably from 3 to 5 minutes, more preferably 4 to 5 minutes, before being rinsed off.

Leave-on conditioners for use in the invention are typically applied to the hair and left on the hair for more than 10 minutes, and preferably are applied to the hair after washing and not rinsed out until the next wash.

### The linear cationic conditioning primary surfactant (i)

Compositions of the invention comprise 0.01 to 10 wt % of a linear cationic conditioning primary surfactant; selected from structure 1 and mixtures thereof 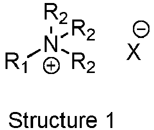 wherein:
- R₁ comprises a linear alkyl chain having a carbon-carbon chain length of from C₁₆ to C₂₄, preferably C₁₈ to C₂₂;
- R₂ comprises a proton or a linear alkyl chain having a carbon-carbon chain length of from C₁ to C₄, preferably C₁ to C₂ or a benzyl group; and
- X is an organic or inorganic anion.

Preferably, the carbon-carbon chain length of R₁ in structure 1 differs from the atom-atom chain length of R₃ in structure 2 by from 3 to 12, more preferably from 4 to 12, even more preferably from 6 to 12, most preferably from 6 to 10 atoms, such that the carbon-carbon chain length of R₁ is structure 1 is longer than the atom-atom chain length of R₃ in structure 2.

In structure 1, the amine head group is charged within the final formulation. Raw materials include, however, species where the charge is not permanent and can be induced by protonation in the formulation using a strong acid. When R₂ is a proton in the above general formulae therefore, the proton may be present in the raw material or become associated during formulation.

Optionally, the alkyl groups may comprise one or more ester (-OCO- or -COO-), amido (-NOC- or NCO-), and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable quaternary amine salts for use in conditioner compositions according to the invention are quaternary amine salt comprising from 12 to 24 carbon atoms, preferably from 16 to 22 carbon atoms.

Suitable quaternary amine salts for use in conditioner compositions according to the invention include cetyltrimethylammonium chloride, behenyltrimethylammonium chloride, Behentrimonium methosulphate, BehenylAmido Propyl Di-Methyl Amine, cetyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, Stearalkonium Chloride, Stearalkonium methosulphate, didodecyldimethylammonium chloride, dioctadecyldimethylammonium chloride, tallowtrimethylammonium chloride. dihydrogenated tallow dimethyl ammonium chloride (e.g., Arquad 2HT/75 from Akzo Nobel) and cocotrimethylammonium chloride.

Preferred quaternary amine salts selected from behenyltrimethylammonium chloride, Behentrimonium methosulphate, cetyltrimethylammonium chloride, and mixtures thereof.

A particularly useful cationic surfactant for use in conditioners according to the invention is cetyltrimethylammonium chloride, available commercially, for example as GENAMIN CTAC, ex Hoechst Celanese. Another particularly preferred cationic surfactant for use in conditioners according to the invention is behenyltrimethylammonium chloride, available commercially, for example as GENAMIN KDMP, ex Clariant.

Further suitable cationic surfactants include those materials having the CTFA designations Quaternium-5, Quaternium-31, and Quaternium-18. Mixtures of any of the foregoing materials may also be suitable.

Another example of a class of suitable cationic surfactants for use in the invention, either alone or together with one or more other cationic surfactants, is a combination of (i) and (ii) below:
(i) an amidoamine corresponding to the general formula (II):

   R¹CONH(CH₂)ₘN(R²)R³ (II)

   in which R¹ is a hydrocarbyl chain having 10 or more carbon atoms, R² and R³ are independently selected from hydrocarbyl chains of from 1 to 10 carbon atoms, and m is an integer from 1 to about 10; and
(ii) an acid.

As used herein, the term hydrocarbyl chain means an alkyl or alkenyl chain.

Preferred amidoamine compounds are those corresponding to formula (I) in which

R¹ is a hydrocarbyl residue having from about 11 to about 24 carbon atoms,

R² and R³ are each independently hydrocarbyl residues, preferably alkyl groups, having from 1 to about 4 carbon atoms, and m is an integer from 1 to about 4.

Preferably, R² and R³ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyldimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include:
stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a
tradename INCROMINE BB available from Croda (North Humberside, England), and various amidoamines with tradenames SCHERCODINE series available from Scher (Clifton New Jersey, USA).

Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than
95 mole% (293 K) of the amidoamine present.

In compositions of the invention, the level of linear cationic conditioning primary surfactant will generally range from 0.01 to 10%, more preferably 0.05 to 7.5%, most preferably 0.1 to 5% by total weight of the composition.

### The linear fatty material (ii)

The composition of the invention comprises from 0.1 to 10 wt % of a linear fatty material.

The combined use of fatty materials and cationic surfactants in conditioning compositions is believed to be especially advantageous, because this leads to the formation of a structured lamellar or liquid crystal phase, in which the cationic surfactant is dispersed.

By "fatty material" is meant a fatty alcohol, an alkoxylated fatty alcohol, a fatty acid or a mixture thereof. Preferably the linear fatty material is selected from a fatty alcohol and a fatty acid, most preferably a fatty alcohol.

Preferably, the alkyl chain of the fatty material is fully saturated. Representative fatty materials comprise from 8 to 22 carbon atoms, more preferably 16 to 22.

Suitable fatty alcohols comprise from 8 to 22 carbon atoms, preferably 16 to 22, most preferably C16 to C18. Fatty alcohols are typically compounds containing straight chain alkyl groups. Preferably, the alkyl groups are saturated. Examples of preferred fatty alcohols include cetyl alcohol, stearyl alcohol and mixtures thereof. The use of these materials is also advantageous in that they contribute to the overall conditioning properties of compositions for use in the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty material in conditioners of the invention is suitably from 0.01 to 10, preferably from 0.1 to 10, and more preferably from 0.1 to 5 percent by weight of the total composition. The weight ratio of cationic surfactant to fatty alcohol is suitably from 10:1 to 1:10, preferably from 4:1 to 1:8, optimally from 1:1 to 1:7, for example 1:3.

### The particulate benefit agent (iii)

The composition of the invention comprises a particulate benefit agent. The particulate benefit agent is selected from conditioning actives, and mixtures thereof. More preferably the particulate benefit agent is selected from silicone emulsions and mixtures thereof.

Preferred conditioning actives are silicone emulsions.

Preferred silicone emulsions do not comprise a hydrophobic modification, preferably the silicone emulsion is not a myristyloxyl modified silicone, most preferably not a myristyloxyl modified silicone or a cetyloxyl modified silicone. Most preferably, the silicone emulsions for use in the compositions of the invention are selected from emulsions of dimethicone, dimethiconol, amodimethicone and mixtures thereof.

Suitable silicones include polydimethylsiloxanes which have the CTFA designation dimethicone. Also suitable for use compositions of the invention are polydimethyl siloxanes having hydroxyl end groups, which have the CTFA designation dimethiconol. Preferably, the silicone is selected from the group consisting of dimethicone, dimethiconol, amodimethicone and mixtures thereof. Also preferred are blends of amino-functionalised silicones with dimethicones.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the compositions of the invention will typically have a D90 silicone droplet size in the composition of less than 30, preferably less than 20, more preferably less than 10 micron, ideally from 0.01 to 1 micron. Silicone emulsions having an average silicone droplet size (D50) of 0.15 micron are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in compositions of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone". A preferred amodimethicone is commercially available from Dow Corning as DC 7134.

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The total amount of silicone is preferably from 0.1 wt % to 10 wt % of the total composition more preferably from 0.1 wt % to 5 wt %, most preferably 0.25 wt % to 3 wt % is a suitable level.

### The linear cationic co-surfactant (iv)

The composition of the invention comprises a linear cationic co-surfactant, according to structure 2: 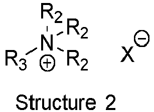 wherein:
- R₂ comprises a proton or a linear alkyl chain having a carbon-carbon chain length of from C₁ to C₄, preferably C₁ to C₂ or a benzyl group;
- R₃ comprises a linear alkyl chain comprising an ester group and having an atom-atom chain length of from 3 to 15, preferably 10 to 14; and
- X is an organic or inorganic anion;

wherein the carbon-carbon chain length of R₁ in structure 1 differs from the atom-atom chain length of R₃ in structure 2 by at least 3 atoms, such that the carbon-carbon chain length of R₁ is structure 1 is longer than the atom-atom chain length of R₃ in structure 2; and
wherein the molar ratio of linear cationic co-surfactant (iv) to linear cationic conditioning primary surfactant (i) is in the range of from 1:20 to 1:1, preferably from 1:10 to 1:1, preferably 1:5 to 1:2.

Preferably, the carbon-carbon chain length of R₁ in structure 1 differs from the atom-atom chain length of R₃ in structure 2 by from 3 to 12, more preferably from 4 to 12, even more preferably from 6 to 12, most preferably from 6 to 10 atoms, such that the carbon-carbon chain length of R₁ is structure 1 is longer than the atom-atom chain length of R₃ in structure 2.

R₃ comprises a linear alkyl chain comprising an ester group and having an atom-atom chain length of from 3 to 15, preferably 3 to 14, more preferably 6 to 14, even more preferably 8 to 14, most preferably 10 to 14 .

The linear co-surfactant is present in an amount of from 0.01 to 5 wt %, preferably 0.1 to 2, more preferably 0.1 to 1.0, most preferably 0.2 to 0.7 wt % based on weight of total composition)

X is an organic or inorganic anion. Preferably, X comprises an anion selected from the halide ions; sulphates of the general formula RSO₃⁻, wherein R is a saturated or unsaturated alkyl radical having 1 to 4 carbon atoms, and anionic radicals of organic acids.

Preferred halide ions are selected from fluoride, chloride, bromide and iodide. Preferred anionic radicals of organic acids are selected from maleate, fumarate, oxalate, tartrate, citrate, lactate and acetate. Preferred sulphates are methanesulphonate and ethanesulphonate.

Most preferably, X⁻ comprises an anion selected from a halide, a methanesulfonate group and an ethanesulphonate group.

In a preferred embodiment,
- R₃ comprises linear alkyl chains, saturated or unsaturated, comprising an ester group and with atom-atom chain lengths of from 10 to 14;
- R₂ comprises a proton or an alkyl chain having a carbon-carbon chain length of from C₁ to C₂; and
- X is selected from a halide, methanesulphonate and ethanesulphonate.

Examples of suitable materials according to structure 2 are N,N,N-trimethyl-2-(octyloxy)-2-oxoethan-1-aminium chloride and N,N,N-trimethyl-2-(octyloxy)-2-oxoethan-1-aminium methanesulfonate, which can be synthesized using a modification of the method outlined in Chemistry, A European Journal, 2008, 14, 382. An acid-catalysed condensation reaction of betaine with octanol furnishes the desired product in one step.

### Composition rheology

The compositions of the invention provide good viscosity and yield stress properties.

The compositions have a preferred yield stress range of from 30 to 200 Pascals (Pa), most preferably from 40 to 150 Pa peak value at 25 °C and 1 Hz. The method to measure the yield stress uses a serrated parallel-plate geometry, 40mm in diameter, attached to a suitable rheometer capable of applying oscillations at a constant frequency of 1Hz, and an amplitude sweep in the range of 0.1% to 2000%. The amplitude sweep range is applied at no more than ten points per decade of strain range covered at no more than 4 cycles per amplitude. The instrument should be operated under controlled strain, such as with the ARES G2 Rheometer from TA Instruments. The geometry's temperature should be set at 25°C by means of, for example, a Peltier-controlled plate, or a recirculating bath. The yield stress is determined by plotting the elastic stress against strain amplitude, and at the peak of the curve, the maximum value is quoted as the yield stress. The elastic stress is calculated as the multiplication of (storage modulus)*(strain amplitude), each readily obtained from the instrument.

The compositions preferably have a viscosity of from 5,000 to 750,000 centipoise, preferably from 50,000 to 600,000 centipoise, more preferably from 50,000 to 450,000 as measured at 30°C on a Brookfield RVT using a Spindle A or B at 0.5 rpm for 60 seconds on a Helipath stand.

A preferred conditioner comprises a conditioning gel phase. These conditioners have little or no vesicle content. Such conditioners and methods for making them are described in WO2014/016354, WO2014/016353, WO2012/016352 and WO2014/016351.

A composition comprising such a conditioning gel phase confers a Draw Mass of from 1 to 250 g, preferably 2 to 100 g, more preferably 2 to 50 g, even more preferably 5 to 40 g and most preferably 5 to 25 g to hair treated with the composition.

Draw Mass is the mass required to draw a hair switch through a comb or brush. Thus the more tangled the hair the greater the mass required to pull the switch through the comb or brush, and the greater the level of condition of the hair, the lower the Draw Mass.

The Draw Mass is the mass required to draw a hair switch, for example of weight 1 to 20 g, length 10 to 30 cm, and width 0.5 to 5 cm through a comb or brush, as measured by first placing the hair switch onto the comb or brush, such that from 5 to 20 cm of hair is left hanging at the glued end of the switch, and then adding weights to the hanging end until the switch falls through the comb or brush.

Preferably, the hair switch is of weight 1 to 20 g, more preferably 2 to 15 g, most preferably from 5 to10 g. Preferably, the hair switch has a length of from 10 to 40 cm, more preferably from 10 to 30 cm, and a width of from 0.5 to 5 cm, more preferably from 1.5 to 4 cm.

Most preferably, the Draw Mass is the mass required to draw a hair switch, for example of weight 10 g, length 20 cm, and width 3 cm through a comb or brush, as measured by first placing the hair switch onto the comb or brush, such that from 20 cm of hair is left hanging at the glued end of the switch, and then adding weights to the hanging end until the switch falls through the comb or brush.

### Further ingredients

The composition according to the invention may comprise any of a number of ingredients which are common to hair conditioning compositions.

Other ingredients may include, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

Preferably, the further ingredients include perfumes, , preservatives, colours and conditioning silicones.

The compositions of the invention are preferably free from viscosity modifiers and thickening agents for example thickening polymers.

Mixtures of any of the above active ingredients may also be used.

Generally, such ingredients are included individually at a level of up to 2%, preferably up to 1 %, by weight of the total composition.

Embodiments of the invention are given in the following examples, in which all percentages are quoted by weight based on total weight unless otherwise stated.

### Examples

### Example 1: Composition 1 in accordance with the invention and Comparative Compositions A and B

The following compositions were prepared:
Example 1, in accordance with the invention, containing a co-surfactant with an ester-containing chain of length 11.

Comparative example A with no co-surfactant material having a linear alkyl chain.

Comparative example B containing a co-surfactant with an ester-containing chain of length 21.

**Table 1: Compositions of examples A and B (comparative) and examples 1 (in accordance with the invention). Amounts shown in wt % by wt of total composition.**

| | Example A | Example B | Example 1 |
|---|---|---|---|
| Ingredient | Comparative | Gel phase incl. 21 atom linear betaine ester chain | Gel phase incl. 11 atom linear betaine ester chain |
| Behentrimonium Chloride | 2.00 | 1.60 | 1.60 |
| Cetearyl Alcohol | 4.00 | 3.20 | 3.20 |
| N,N,N-trimetyl-2-(octanoyloxy)-2-oxoethan-1-aminium methanesulfonate | - | 0.47 | - |
| N,N,N-trimethyl-2-(octyloxy)-2-oxoethan-1-aminium chloride | - | - | 0.27 |
| Dimethicone 600K and Amodimethicone 2000nm | 1.00 | 1.00 | 1.00 |
| Perfume | 0.60 | 0.60 | 0.60 |
| Preservative | 0.1 | 0.1 | 0.1 |
| Water | to 100 | to 100 | to 100 |

The conditioners in examples 1, A and B were prepared using the following method:
1. Surfactants and fatty materials were added to a suitable vessel and heated to above the melting point of the fatty materials.
2. The molten blend was added to a suitable amount of water according to the compositions in Table 1, at a temperature of between room temperature and below the melting point of the fatty materials.
3. The mixture was mixed until opaque and thick.
4. The heat was then turned off, cooled to room temperature, and the rest of the water was added along with the remaining materials.
5. Finally, the formulation was mixed at high shear using a suitable homogenising device.

### Example 2: Treatment of hair with compositions A and B and 1

The hair used was dark brown European hair, in switches of 5 g weight and 6 inches in length.

Hair was first treated with a cleansing shampoo using the following method:-
The hair fibres were held under running water for 30 seconds, shampoo applied at a dose of 0.1 ml of shampoo per 1g of hair and rubbed into the hair for 30 seconds. Excess lather was removed by holding under running water for 30 seconds and the shampoo stage repeated. The hair was rinsed under running water for 1 minute.

The wet hair was then treated with the compositions using the following method:-Conditioner was applied to the wet hair at a dose of 0.2 ml of conditioner per 1g of hair and massaged into the hair for 1 minute. The hair was rinsed under running water for 1 minute and excess water removed.

### Example 3: Silicone Deposition onto hair treated with Compositions 1, A and B

The amount of silicone deposited onto hair was quantified using x-ray fluorescence (XRF).

**Table 2: Amount of silicone deposited on hair treated with examples 1, A and B.**

| | Example A | Example B | Example 1 |
|---|---|---|---|
| Ingredient | Comparative | Gel phase incl. A21 linear betaine ester quat. | Gel phase incl. A11 linear betaine ester quat |
| Silicone Deposition [ppm] | 499 | 486 | 602 |
| Silicone Deposition ST DEV [ppm] | 80 | 68 | 70 |

It will be seen that the composition in accordance with the example results in dramatically higher levels of silicone deposition onto hair, despite containing only about half the amount of co-surfactant and Comparative B.

## Claims

1. A composition comprising:
(i) 0.01 to 10 wt % of a linear cationic conditioning primary surfactant; selected from structure 1 and mixtures thereof: 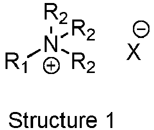 wherein:
• R₁ comprises a linear alkyl chain having a carbon-carbon chain length of from C₁₆ to C₂₄, preferably C₁₈ to C₂₂;
• R₂ comprises a proton or a linear alkyl chain having a carbon-carbon chain length of from C₁ to C₄, preferably C₁ to C₂ or a benzyl group; and
• X is an organic or inorganic anion;
(ii) 0.1 to 10 wt % of a linear fatty material;
(iii) a particulate benefit agent selected from conditioning actives and mixtures thereof; and
(iv) 0.01 to 5 wt % of a linear cationic co-surfactant, selected from structure 2 and mixtures thereof 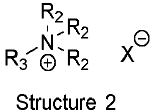 wherein:
• R₂ comprises a proton or a linear alkyl chain having a carbon-carbon chain length of from C₁ to C₄, preferably C₁ to C₂ or a benzyl group;
• R₃ comprises a linear alkyl chain comprising an ester group and having an atom-atom chain length of from 3 to 15, preferably 10 to 14; and
• X is an organic or inorganic anion;
wherein the carbon-carbon chain length of R₁ in structure 1 differs from the atom-atom chain length of R₃ in structure 2 by at least 3 atoms, such that the atom-atom chain length of R₁ in structure 1 is longer than the atom-atom chain length of R₃ in structure 2; and
wherein the molar ratio of linear cationic co-surfactant (iv) to linear cationic conditioning primary surfactant (i) is in the range of from 1:20 to 1:1.

2. A composition as claimed in claim 1, wherein the carbon-carbon chain length of R₁ in structure 1 differs from the atom-atom chain length of R₃ in structure 2 by from 3 to 12, preferably from 6 to 10 atoms, such that the carbon-carbon chain length of R₁ is structure 1 is longer than the atom-atom chain length of R₃ in structure 2.

3. A composition as claimed in claim 1 or claim 2, wherein R₃ comprises a linear alkyl chain having an atom-atomchain length of from 6 to 14, preferably 10 to 14.

4. A composition as claimed in any preceding claim, wherein the linear cationic conditioning primary surfactant is selected from behenyltrimethylammonium chloride, behentrimonium methosulphate, cetyltrimethylammonium chloride, and mixtures thereof.

5. A composition as claimed in any preceding claim, wherein the conditioning actives are silicone emulsions.

6. A composition as claimed in claim 5, wherein the silicone emulsions are selected from emulsions of dimethicone, dimethiconol, amodimethicone and mixtures thereof.

7. A composition as claimed in claim 5 or claim 6, wherein the silicone emulsion is present in an amount of from 0.1 wt % to 10 wt % of the total composition most preferably 0.25 wt % to 3 wt %.

8. A composition as claimed in any preceding claim, wherein the linear cationic co-surfactant is present in an amount of from 0.1 to 2 wt %, most preferably 0.2 to 0.7 wt %.

9. A composition as claimed in any preceding claim, wherein the molar ratios of linear cationic co-surfactants (iv) to linear cationic surfactants (i) are in the range of from 1:10 to 1:1, preferably 1:5 to 1:2.

10. A composition as claimed in any preceding claim, which has a viscosity of from 5,000 to 750,000 centipoise, (5 to 750 kg/m-s) as measured at 30°C on a Brookfield RVT using a Spindle A or B at 0.5 rpm for 60 seconds on a Helipath stand.

11. A composition as claimed in any preceding claim, wherein X⁻ comprises an anion selected from a halide, a methanesulfonate group and an ethanesulphonate group.

12. A method of increasing deposition of a particulate benefit agent selected from conditioning actives, to hair comprising the steps of applying to hair a composition as defined in any of claims 1 to 11 and rinsing the hair with water.

## Patentansprüche

1. Zusammensetzung, umfassend:
(i) 0,01 bis 10 Gew.-% eines linearen, kationischen, konditionierenden primären Tensids, ausgewählt aus der Struktur 1 und Mischungen davon: 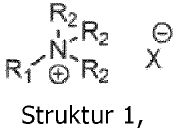 worin:
• R₁ eine lineare Alkylkette mit einer Kohlenstoff-Kohlenstoff-Kettenlänge von C₁₆ bis C₂₄, vorzugsweise von C₁₈ bis C₂₂, umfasst;
• R₂ ein Proton oder eine lineare Alkylkette mit einer Kohlenstoff-Kohlenstoff-Kettenlänge von C₁ bis C₄, vorzugsweise von C₁ bis C₂, oder eine Benzylgruppe umfasst; und
• X ein organisches oder anorganisches Anion ist;
(ii) 0,1 bis 10 Gew.-% eines linearen Fettmaterials;
(iii) ein teilchenförmiges Pflegemittel, ausgewählt aus konditionierenden Wirkstoffen und Mischungen davon; und
(iv) 0,01 bis 5 Gew.-% eines linearen, kationischen Co-Tensids, ausgewählt aus der Struktur 2 und Mischungen davon 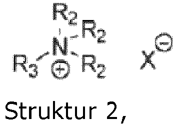 worin
• R₂ ein Proton oder eine lineare Alkylkette mit einer Kohlenstoff-Kohlenstoff-Kettenlänge von C₁ bis C₄, vorzugsweise von C₁ bis C₂, oder eine Benzylgruppe umfasst;
• R₃ eine lineare Alkylkette umfasst, die eine Estergruppe umfasst und eine Atom-Atom-Kettenlänge von 3 bis 15, vorzugsweise von 10 bis 14, aufweist; und
• X ein organisches oder anorganisches Anion ist;
wobei sich die Kohlenstoff-Kohlenstoff-Kettenlänge von R₁ in der Struktur 1 von der Atom-Atom-Kettenlänge von R₃ in der Struktur 2 durch mindestens 3 Atome unterscheidet, so dass die Atom-Atom-Kettenlänge von R₁ in der Struktur 1 länger als die Atom-Atom-Kettenlänge von R₃ in der Struktur 2 ist; und
wobei das Molverhältnis des linearen, kationischen Co-Tensids (iv) zum linearen, kationischen konditionierenden primären Tensid (i) in dem Bereich von 1:20 bis 1:1 liegt.

2. Zusammensetzung, wie im Anspruch 1 beansprucht, wobei sich die Kohlenstoff-Kohlenstoff-Kettenlänge von R₁ in der Struktur 1 von der Atom-Atom-Kettenlänge von R₃ in der Struktur 2 durch 3 bis 12, vorzugsweise durch 6 bis 10 Atome, unterscheidet, so dass die Kohlenstoff-Kohlenstoff-Kettenlänge von R₁ in der Struktur 1 länger als die Atom-Atom-Kettenlänge von R₃ in der Struktur 2 ist.

3. Zusammensetzung, wie in Anspruch 1 oder Anspruch 2 beansprucht, wobei R₃ eine lineare Alkylkette mit einer Atom-Atom-Kettenlänge von 6 bis 14, vorzugsweise von 10 bis 14, umfasst.

4. Zusammensetzung, wie in einem vorhergehenden Anspruch beansprucht, wobei das lineare, kationische, konditionierende primäre Tensid aus Behenyltrimethylammoniumchlorid, Behentrimonium-Methosulfat, Cetyltrimethylammoniumchlorid und Mischungen davon ausgewählt wird.

5. Zusammensetzung, wie in einem vorhergehenden Anspruch beansprucht, wobei die konditionierenden Wirkstoffe Silikonemulsionen sind.

6. Zusammensetzung, wie im Anspruch 5 beansprucht, wobei die Silikonemulsionen aus Emulsionen von Dimethicon, Dimethiconol, Amodimethicon und Mischungen davon ausgewählt sind.

7. Zusammensetzung, wie im Anspruch 5 oder Anspruch 6 beansprucht, wobei die Silikonemulsion in einer Menge von 0,1 Gew.-% bis 10 Gew.-% der Gesamtzusammensetzung, höchst bevorzugt von 0,25 Gew.-% bis 3 Gew.-%, vorliegt.

8. Zusammensetzung, wie in irgendeinem vorhergehenden Anspruch beansprucht, wobei das lineare, kationische Co-Tensid in einer Menge von 0,1 bis 2 Gew.-%, höchst bevorzugt von 0,2 bis 0,7 Gew.-%, vorliegt.

9. Zusammensetzung, wie in einem vorhergehenden Anspruch beansprucht, wobei die Molverhältnisse der linearen, kationischen Co-Tenside (iv) zu den linearen, kationischen Tensiden (i) in dem Bereich von 1:10 bis 1:1, vorzugsweise von 1:5 bis 1:2, liegen.

10. Zusammensetzung, wie in einem vorhergehenden Anspruch beansprucht, die eine Viskosität von 5.000 bis 750.000 centipoise (5 bis 750 kg/m.s) aufweist, gemessen bei 30°C mit einem Brookfield RVT unter Verwendung einer Spindel A oder B bei 0,5 U/min über 60 Sekunden auf einem Helipath-Ständer.

11. Zusammensetzung, wie in einem vorhergehenden Anspruch beansprucht, wobei X⁻ ein Anion umfasst, ausgewählt aus einem Halogenid, einer Methansulfonatgruppe und ein Ethansulfonatgruppe.

12. Verfahren zur Erhöhung der Ablagerung eines teilchenförmigen Pflegemittels, ausgewählt aus konditionierenden Wirkstoffen, auf dem Haar, umfassend die Schritte des Auftragens einer Zusammensetzung, wie in einem der Ansprüche 1 bis 11 definiert, auf das Haar und Spülen des Haars mit Wasser.

## Revendications

1. Composition comprenant :
(i) 0,01 à 10 % en masse d'un tensioactif primaire de conditionnement cationique linéaire ; choisi parmi la structure 1 et des mélanges de celle-ci : 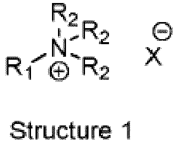 dans laquelle :
• R₁ comprend une chaîne alkyle linéaire ayant une longueur de chaîne carbone-carbone de C₁₆ à C₂₄, de préférence C₁₈ à C₂₂ ;
• R₂ comprend un proton ou une chaîne alkyle linéaire ayant une longueur de chaîne carbone-carbone de C₁ à C₄, de préférence C₁ à C₂ ou un groupe benzyle ; et
• X est un anion organique ou inorganique ;
(ii) 0,1 à 10 % en masse d'un matériau gras linéaire ;
(iii) un agent bénéfique particulaire choisi parmi des actifs de conditionnement et mélanges de ceux-ci ; et
(iv) 0,01 à 5 % en masse d'un co-tensioactif cationique linéaire, choisi parmi la structure 2 et des mélanges de celle-ci 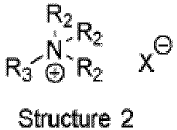 dans laquelle :
• R₂ comprend un proton ou une chaîne alkyle linéaire ayant une longueur de chaîne carbone-carbone de C₁ à C₄, de préférence C₁ à C₂ ou un groupe benzyle ;
• R₃ comprend une chaîne alkyle linéaire comprenant un groupe ester et ayant une longueur de chaîne atome-atome de 3 à 15, de préférence 10 à 14 ; et
• X est un anion organique ou inorganique ;
dans laquelle la longueur de chaîne carbone-carbone de R₁ dans la structure 1 est différente de la longueur de chaîne atome-atome de R₃ dans la structure 2 d'au moins 3 atomes, de sorte que la longueur de chaîne atome-atome de R₁ dans la structure 1 est plus longue que la longueur de chaîne atome-atome de R₃ dans la structure 2 ; et
dans laquelle le rapport molaire de co-tensioactif cationique linéaire (iv) à tensioactif primaire de conditionnement cationique linéaire (i) se trouve dans l'intervalle de 1:20 à 1:1.

2. Composition selon la revendication 1, dans laquelle la longueur de chaîne carbone-carbone de R₁ dans la structure 1 est différente de la longueur de chaîne atome-atome de R₃ dans la structure 2 de 3 à 12, de préférence de 6 à 10 atomes, de sorte que la longueur de chaîne carbone-carbone de R₁ dans la structure 1 est plus longue que la longueur de chaîne atome-atome de R₃ dans la structure 2.

3. Composition selon la revendication 1 ou revendication 2, dans laquelle R₃ comprend une chaîne alkyle linéaire ayant une longueur de chaîne atome-atome de 6 à 14, de préférence de 10 à 14.

4. Composition selon l'une quelconque des revendications précédentes, dans laquelle le tensioactif primaire de conditionnement cationique linéaire est choisi parmi le chlorure de béhényltriméthylammonium, méthosulfate de béhentrimonium, chlorure de cétyltriméthylammonium, et des mélanges de ceux-ci.

5. Composition selon l'une quelconque des revendications précédentes, dans laquelle les actifs de conditionnement sont des émulsions de silicone.

6. Composition selon la revendication 5, dans laquelle les émulsions de silicone sont choisies parmi des émulsions de diméthicone, diméthiconol, amodiméthicone et des mélanges de ceux-ci.

7. Composition selon la revendication 5 ou revendication 6, dans laquelle l'émulsion de silicone est présente dans une quantité de 0,1 % en masse à 10 % en masse de la composition totale encore mieux 0,25 % en masse à 3 % en masse.

8. Composition selon l'une quelconque des revendications précédentes, dans laquelle le co-tensioactif cationique linéaire est présent dans une quantité de 0,1 à 2 % en masse, encore mieux de 0,2 à 0,7 % en masse.

9. Composition selon l'une quelconque des revendications précédentes, dans laquelle les rapports molaires de co-tensioactifs cationiques linéaires (iv) aux tensioactifs cationiques linéaires (i) se trouvent dans l'intervalle de 1:10 à 1:1, de préférence 1:5 à 1:2.

10. Composition selon l'une quelconque des revendications précédentes, qui présente une viscosité de 5 000 à 750 000 centipoises, (5 à 750 kg/m-s) comme mesurée à 30°C sur un RVT Brookfield utilisant une broche A ou B à 0,5 tr/min pendant 60 secondes sur un banc Helipath.

11. Composition selon l'une quelconque des revendications précédentes, dans laquelle X⁻ comprend un anion choisi parmi un halogénure, un groupe méthanesulfonate et un groupe éthanesulfonate.

12. Procédé d'augmentation du dépôt d'un agent bénéfique particulaire choisi parmi des actifs de conditionnement, sur des cheveux comprenant les étapes d'application aux cheveux d'une composition selon l'une quelconque des revendications 1 à 11 et de rinçage des cheveux avec de l'eau.
